# EUROPEAN PATENT SPECIFICATION

(11) **EP 3 395 366 B1**
(45) Date of publication and mention of the grant of the patent: **22.11.2023**
(21) Application number: 16877724.1
(22) Date of filing: 21.12.2016
(51) Int. Cl.: A61K 45/06, A61K 38/20, C07K 19/00, A61P 35/00

(54) **DRUG DESIGN METHOD, OBTAINED DRUG AND APPLICATION THEREOF**
WIRKSTOFFDESIGNVERFAHREN, ERHALTENER WIRKSTOFF UND ANWENDUNG DAVON
PROCÉDÉ DE CONCEPTION DE MÉDICAMENT, MÉDICAMENT OBTENU ET APPLICATION ASSOCIÉE

(30) Priority: 21.12.2015 CN 201510958988; 21.12.2015 CN 201510959364
(43) Date of publication of application: 31.10.2018
(73) Proprietor: Hefei Lifeon Pharmaceutical Co., Ltd., Hefei, Anhui 230088 (CN)
(72) Inventor: ZHANG, Mei, Hefei, Anhui 230088 (CN); JI, Junqiu, Hefei, Anhui 230088 (CN); GAO, Meihua, Hefei, Anhui 230088 (CN); CHEN, Jun, Hefei, Anhui 230088 (CN)
(74) Representative: Nederlandsch Octrooibureau
(86) International application number: PCT/CN2016/111264
(87) International publication number: WO 2017/107914

(56) References cited:
- EP-A1- 2 085 095
- EP-A1- 3 378 947
- EP-B1- 2 256 134
- WO-A1-2014/194784
- CN-A- 103 124 788
- MENRAD A ET AL: "ED-B FIBRONECTIN AS A TARGET FOR ANTIBODY-BASED CANCER TREATMENTS", EXPERT OPINION ON THERAPEUTIC TAR, INFORMA HEALTHCARE, UK, vol. 9, no. 3, 1 June 2005 (2005-06-01), pages 491-500, XP009052148, ISSN: 1472-8222, DOI: 10.1517/14728222.9.3.491

## Description

### Technical Field

The invention relates to the field of drug design and development as well as of pharmaceutical preparations, in particular to the field of design and development of drugs used for diseases related to ED-B, and also to the drugs and use thereof in therapy.

### Background Art

The organs, tissues, cells, bio-macromolecules and their specific drug binding sites where drugs exert effects after entering into the body are called target organs, target tissues, target cells and target sites, respectively. Targets can be proteins, nucleic acids or other substances, including bio-macromolecules such as gene sites, receptors, enzymes, ion channels, nucleic acids and the like, and may generally have important physiological or pathological functions. A drug can specifically act on a target, but some targets of drugs are distributed on organs or tissues other than those the drugs expect to act on, or shed off in the blood, and may competitively bind to the drug, resulting in that part of the drug does not bind to the targets on the target organ, which would affect the effectiveness of the drug, which will on one hand consume the drug and on the other hand produce toxic and side effects. In addition, free target proteins shed off from a tissue in the blood or interstitial fluid may affect the pharmacokinetics of a drug. The above characteristics of targets may lead to a series of problems, such as increased dosage, great individual differences, increased toxic and side effects of drug, ineffectiveness of a drug having a narrow therapy window or generation of intolerable toxicity etc.

During the development of an antibody or antibody-related drug, the side effects caused by off-target of the drug and non-target organ targets are even more obvious. Although antibody drugs against tumors with great expectations bring hope to tumor patients, their effects are still not ideal. For example, an antibody drug developed against human epidermal growth factor receptor 2 (HER2), e.g. Herceptin, have achieved favorable effects in breast cancer treatment. However, due to the distribution of HER-2 proteins on the myocardial transverse tubules, long-term use of such drug may increase cardiovascular toxicity and lead to congestive heart failure, and light, even moderate to severe heart failures (Nemeth, B. T., et al. (2016). Br J Pharmacol.doi: 10.1111/bph.13643). This is a side effect caused by low specificity of the organ where the target proteins of the drug are distributed. An antibody-drug conjugate (ADC) targeting the HER2, such as ado-trastuzumab emtansine (T-DM1), carries an efficient cytotoxic agent and is capable of inhibiting tubulin polymerization and microtubule dynamics. However, this drug may also lead to cardio-toxicity due to the distribution of HER2 in the heart tissue. In addition, the metabolism of the T-DM1 in non-lesion sites may produce hepatotoxicity (Yan, H., et al. (2016). Mol Cancer Ther 15(3): 480-490). An antibody drug developed against Extra Domain B (ED-B) of Fibronectin, such as L19-IL2, does not produce remarkable pharmaceutical effect by the way of intravenous administration (Eigentler, T.K., et al., (2011) Clin Cancer Res, 17(24): 7732-42), but produces a significant effect by local delivery to tumor lesions (Weide, B., et al., (2014) Cancer immunology research 2(7): 668-678).

Menrad et al. (DOI: 10.1517/14728222.9.3.491) describes L19-IL2, which is a fusion protein comprising the ED-B antibody L19 as targeting moiety and IL-2 as biologically active moiety, for use in the treatment of solid tumors, namely pancreas carcinoma and hepatocellular carcinoma. EP 2 085 095 A1 discloses that ED-B is strongly expressed in the solid tumor U87 glioblastoma, and also in Ramos lymphoma; it further describes the effectiveness of L19-IL2, which is a fusion protein comprising the ED-B antibody LI 9 as targeting moiety and IL-2 as biologically active moiety, against lymphoma.

### Summary of the Invention

In one aspect, the invention provides a kit for use in treating a solid tumor associated with ED-B in a subject, wherein the solid tumor associated with ED-B has presence and/or expression of ED-B, the kit comprising a biologically inert drug and a biologically active drug, wherein
- the biologically active drug comprises a targeting moiety and a biologically active moiety;
- the biologically inert drug comprises a targeting moiety and a biologically inert moiety;
optionally the biologically inert drug and the biologically active drug have the same targeting moiety, wherein the biologically inert drug and the biologically active drug are mixed and prepared into a single preparation or composition, or the biologically inert drug and the biologically active drug are prepared into separate preparations or compositions, respectively, wherein the targeting moiety is an antibody or a binding fragment thereof against the Extra-domain B (ED-B) of fibronectin, wherein the biologically active moiety is selected from TNF-alpha, IL-2 or IL-12, wherein the biologically inert moiety is selected from the group consisting of an antibody constant region, albumin, and a polyethylene glycol, and wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug.

In a second aspect the invention provides a pharmaceutical composition for use in treating a solid tumor associated with ED-B in a subject, wherein the solid tumor associated with ED-B has presence and/or expression of ED-B, the composition comprising a biologically inert drug, a biologically active drug, and optionally a pharmaceutically acceptable carrier and/or excipient, wherein
- the biologically active drug comprises a targeting moiety and a biologically active moiety;
- the biologically inert drug comprises a targeting moiety and a biologically inert moiety;

optionally the biologically inert drug and the biologically active drug have the same targeting moiety,
wherein the targeting moiety is an antibody or a binding fragment thereof against the Extra-domain B (ED-B) of fibronectin, wherein the biologically active moiety is selected from TNF-alpha, IL-2 or IL-12, wherein the biologically inert moiety is selected from the group consisting of an antibody constant region, albumin, and a polyethylene glycol, and wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug.

In some embodiments of the first or second aspect the biologically active moiety is cytokine IL-2 or a peptide set forth in SEQ ID NO: 9, and/or the biologically active drug is a polypeptide set forth in SEQ ID NO: 6.

In a third aspect the invention provides a biologically inert drug for use in treating a solid tumor associated with ED-B in a subject, wherein the solid tumor associated with ED-B has presence and/or expression of ED-B, the biologically inert drug comprising a targeting moiety targeting a target of interest and a biologically inert moiety, wherein the targeting moiety is an antibody or a binding fragment thereof against the Extra-domain B (ED-B) of fibronectin, and the biologically inert moiety is selected from the group consisting of an antibody constant region, albumin, and a polyethylene glycol, wherein the treatment comprises administering a biologically active drug comprising a targeting moiety and a biologically active moiety, and wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug, wherein the targeting moiety of the biologically active drug is an antibody or a binding fragment thereof against the Extra-domain B (ED-B) of fibronectin, and wherein the biologically active moiety of the biologically active drug is selected from TNF-alpha, IL-2 or IL-12.

In some embodiments of any of the first, second, or third aspects, the targeting moiety is selected from the group consisting of an intact antibody, an antibody CDR, an antibody variable region, a Fab fragment, a Fab' fragment, a F(ab)'₂ fragment, a single-chain FV (scFV), a FV fragment, a light chain of an antibody, a heavy chain of antibody, a single-domain antibody, a diabody and a linear antibody. In some embodiments, the biologically inert moiety is IgG4 Fc region. In some embodiments the targeting moiety is selected from the group consisting of CGS-1, CGS-2, L19, B5 antibody and an ED-B binding fragment thereof, wherein the ED-B binding fragment is selected from the group consisting of an antibody CDR, an antibody variable region, a Fab fragment, a Fab' fragment, a F(ab)'₂ fragment, a single-chain FV (scFV), a FV fragment, a light chain of an antibody, a heavy chain of an antibody, a single-domain antibody, a diabody and a linear antibody. In some embodiments the biologically inert moiety is a peptide set forth in SEQ ID NO: 4, and/or the biologically inert drug is a polypeptide set forth in SEQ ID NO: 5 or a dimer thereof. In other embodiments the targeting moiety is a peptide set forth in SEQ ID NO: 3.

In a fourth aspect the invention provides a biologically inert drug comprising a targeting moiety targeting a target of interest and a biologically inert moiety, wherein the targeting moiety is an antibody or a binding fragment thereof against the Extra-domain B (ED-B) of fibronectin, and the biologically inert moiety is selected from the group consisting of an antibody constant region, albumin, and a polyethylene glycol,
wherein the biologically inert moiety is a polypeptide set forth in SEQ ID NO: 4, and/or the biologically inert drug is a polypeptide set forth in SEQ ID NO: 5 or a dimer thereof,
wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug.

### Brief Description of the Drawings

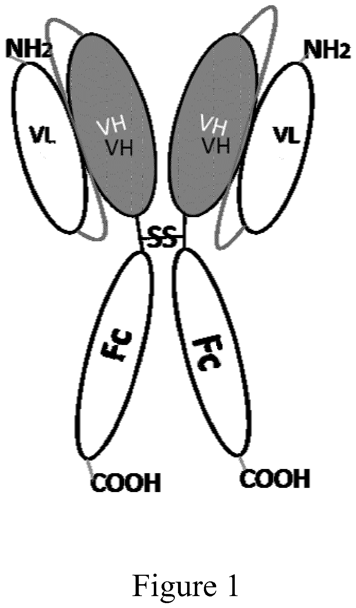

FIG. 1 is a schematic diagram of B5-Fc fusion protein dimer.

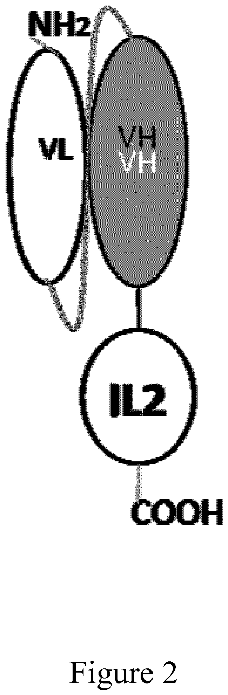

FIG. 2 is a schematic diagram of B5-IL2 fusion protein.
FIG. 3 illustrates expression of ED-B in subject's blood.

### Detailed Description of the Invention

Unless defined otherwise, all technical and scientific terms used herein will have the same meaning as commonly known to those of ordinary skill in the art. In addition, unless otherwise required particularly, singular terms shall include pluralities and plural terms shall include the singular. The techniques and methods described above are generally carried out according to conventional methods well known in the art and as described in reference documents cited herein. See, for example, Sambrook et al. Molecular Cloning: A Laboratory Manual (3rd ed., Cold Spring Harbor Laboratory Press, Cold Spring Harbor, N. Y. (2001)).

The disclosure relates to a method of developing a drug system which improves the drug specificity, a corresponding drug and a pharmaceutical composition, a kit or a cartridge, and a method for treating related diseases. According to the invention, the specificity of a drug is further improved, and the distribution of the drug at a lesion organ or tissue is increased while the distribution of the drug at normal tissues is reduced. Thus, the damage to non-target organs or non-target tissues can be reduced, and the therapeutic efficacy of the drug can be significantly improved with reduced drug waste.

In one aspect, the invention achieves accurate administration by blocking or eliminating the targets of the tissues which are not intended to be targeted by the administration. The development of a high-specificity drug system, a drug and a formulation according to the invention is to design a new drug substantially having no biological activity to be distributed on non-lesion targets to replace part of a biologically active drug, so as to reduce the dosage of the biologically active drug or increase the distribution proportion of the biologically active drug in lesion sites, thereby reducing the toxic and side effects of the drug or improving the efficacy of the drug. In another aspect, the high-specificity drug system, the drug and the formulation according to the invention may also be used for the target mainly or merely present in diseased sites, by administering a biologically inert drug first to distribute on expressed lesion targets to replace a biologically active drug, and then administering the biologically active drug to distribute on newly generated lesion targets.

The drug according to the invention may be a nucleic acid drug, a polypeptide drug, a protein drug, or a conjugate of a biological drug and a chemical drug. Organs, tissues, cells, bio-macromolecules and specific drug binding sites thereof where drugs are able to exert activities in human body are called target organs, target tissues, target cells, target molecules and targets, respectively. Target molecules may be proteins, nucleic acids or other substances, including bio-macromolecules such as gene sites, receptors, enzymes, ion channels, nucleic acids and the like, and generally have important physiological or pathological functions.

The "blocking or eliminating" a drug target on a non-target organ according to the invention may be partial blocking or partial eliminating, and may produce a certain blocking or eliminating effect on sites such as target organs, target tissues, lesions and the like. Since the distribution density or concentration of drug targets at a lesion site is higher than that in normal tissues and drug targets are less distributed on normal tissues or difficult to detect, or free targets in the blood are more likely to bind to a drug than targets in tissues, the administered biologically inert drug may have more significant effect on the targets in normal tissues or in the blood than on the targets in lesions.

The high-specificity drug system according to the invention comprises a biologically inert drug and a biologically active drug, wherein the biologically inert drug and the biologically active drug are able to target the same target, for example, have the same targeting moiety. A high-specificity drug system development method according to the disclosure also includes a drug design method, a design method of a drug chaperone, a method of using combined drug and drug chaperone. These methods may be used individually, and in combination.

The biological activity according to the invention refers to a biological effect or therapeutic effect of biological metabolism or physiological regulatory function, such as activities of promoting digestion and absorption, promoting immune regulation, regulating hormone, anti-bacteria, anti-virus, anti-tumor, cytotoxicity, reducing blood pressure, reducing blood fat, and the like.

The wordings "biologically active drug" and "drug having biological activity" according to the invention have the same meaning, referring to a substance having biological activity in an organism, especially a human body, which is capable of interacting with cells, proteins and the like in the human body and affecting its physiological, biochemical or pathological process, thereby preventing, diagnosing or treating a disease.

The "biological inert" according to the invention means there is no biological activity or weak biological activity as described above. The wordings "biologically inert drug" and "drug having biological inertia" according to the invention have the same meaning, referring to a substance substantially having no biological activity in an organism, especially a human body, which although is capable of binding to cells, proteins and the like in human body, but cannot significantly influence or may have a weak effect on the physiological, biochemical or pathological process in the body, and is mainly used for the purposes of assisting the biologically active drug to improve pharmaceutical efficacy or reduce toxic and side effects, etc. The biologically inert drug may also produce a certain biological effect, such as weak ADCC activity or CDC activity, but the biologically inert drug has different pharmaceutical activities compared to the biologically active drug, or the pharmaceutical activity of the biologically inert drug is significantly lower than that of the biologically active drug.

A biologically inert drug substantially has no biological activity relative to a biologically active drug, which mainly means that the biologically inert drug does not have the pharmaceutical effect as the biologically active drug, or at the same administered dosage, its pharmaceutical effect may be neglected compared with the biologically active drug. It does not exclude that the biologically inert drug may have other functions or physiological activities, e.g. the biologically inert drug may have fluorescence, be capable of blocking the binding of a target to the biologically active drug, and be capable of binding to other ligands or receptors. In particular, the biologically inert drug does not have the biological activity of the corresponding active drug, or does not have a therapeutic effect when used alone. In the invention, the biologically inert drug is used along with the biologically active drug, and thus can also be referred to as a drug chaperone. The "drug chaperone" used herein refers to an auxiliary agent or an auxiliary molecule which recognizes the same target as an active drug and is used along with the active drug, i.e., the biologically inert drug according to the invention.

A biologically active drug can recognize and bind to a specific target, thereby exerting the therapeutic effect of the drug. The active moiety (e.g. a bioactive molecule) of the biologically active drug can produce a desired therapeutic effect, and may be, for example, a cell toxin, a cytokine, a radioactive isotope, a chemical drug, a constant region of an antibody having biological activities, or a cell having biological activities, such as T lymphocyte.

As used herein, "treatment" refers to a process of obtaining a beneficial or desired outcome including a clinical outcome. For the invention, the beneficial or desirable clinical outcome includes, but not limited to, one or more of the following: relieving one or more symptoms caused by a disease; reducing the severity of a disease, stabilizing a disease (e.g., preventing or delaying disease deterioration), preventing or delaying disease propagation (e.g. metastasis), preventing or delaying disease recurrence, delaying or slowing down disease progression, improving disease status, alleviating (part of or all) disease, reducing the dosage of one or more other drugs needed for the treatment of disease, delaying disease progression, enhancing or improving life quality, increasing body weight, and/or prolonging survival. The "treatment" also encompasses reduction of a pathological result of cancer (e.g. tumor volume).

In one aspect, the disclosure relates to a method for designing and developing a drug, comprising: based on a target, selecting a targeting moiety specifically binding to the target, producing a drug having biological activity based on the targeting moiety to exert therapeutic effects of the drug, and simultaneously producing a drug having biological inertia based on the targeting moiety or the target to exert a target shielding effect or exert or promote target clearance, such as target degradation, endocytosis of the target by cells, and entering of the target into the digestive system and so on.

In an embodiment, the invention achieves the accurate administration to a lesion by blocking or eliminating targets (e.g., proteins) in non-lesions such as normal tissues or the blood. There are a plurality of methods for shielding and eliminating targets, e.g. using a composition or a formula of a drug having biological inertia and a drug having biological activity, wherein the drug having biological inertia and the drug having biological activity are able to target the same target. The drug having biological activity can recognize and bind to a specific target, thereby producing a therapeutic effect, while the drug having biological inertia can recognize and bind to a specific target, and shield the target or direct the target to degrade, thereby reducing the target capable of binding to the biologically active drug, for example, especially reducing the target distributed in normal tissues capable of binding to the biologically active drug, and also reducing the targets distributed in the blood capable of binding to the biologically active drug.

Alternatively, a biologically active drug and a biologically inert drug are used, wherein the biologically active drug can exert a biological therapeutic effect, while the biologically inert drug can exert an effect of blocking or eliminating targets (e.g., target protein) on a non-target organ, wherein the eliminating targets may be a process of protein hydrolysis, or of accelerating the endocytosis of the target by cells.

In the invention, the biologically inert drug and the biologically active drug may have the same mother nucleus, have the same targeting moiety for example recognizing the same epitope, compete for the same target, or compete for the same protein or protein complex.

In an embodiment, the disclosure provides a drug design method, comprising:
- selecting a targeting moiety which specifically binds to a target of interest;
- linking the targeting moiety with a biologically active moiety, directly covalently or indirectly via a linker, to obtain a biologically active drug; and/or
- linking the targeting moiety with a biologically inert moiety, directly covalently or indirectly via a linker, to obtain a biologically inert drug.

The target in the invention can be a biomolecule expressed or present on a specific diseased organ, tissue and/or cell, such as a receptor, an ion channel or any other suitable surface molecules. In an embodiment, the target may be a specific biomarker for a disease. In an embodiment, the target is not only expressed or present on a diseased organ, tissue and/or cell, but also expressed or present on a normal organ, tissue and/or cell or present in body fluid such as blood. In an embodiment, the target in the invention may include: for example, a receptor tyrosine kinase targets capable of promoting neoangiogenesis, such as epidermal growth factor receptor (EGFR), vascular endothelial cell growth factor receptor (VEGFR), platelet-derived growth factor receptor (PDGFR), fibroblast growth factor receptor (FGFR) and the like, which are also expressed in part of normal tissues or vascular walls; a protein or a tumor marker which is specifically highly expressed in the cell membrane or extracellular matrix of e.g. a tumor or inflammatory tissue, such as extra domain A (ED-A) of fibronectin (FN), carcinoembryonic antigen (CEA), a glycoprotein antigen (such as MUC16/CA125, MUC1/CA 15-3), epithelial cell adhesion molecule (EpCAM) and carcinoembryonic antigen-related cell adhesion molecule (CEACAM1), and the like.

In an embodiment, the target according to the invention includes, but not limited to, HER2, ED-B, ED-A, EGFR, VEGFR, PDGFR, FGFR, CEA, glycoprotein antigens (such as MUC16/CA125, MUC1/CA 15-3), EpCAM and CEACAM1.

The targeting moiety according to the invention is a molecule or a moiety capable of specifically targeting (e.g., recognizing and/or binding to) a target of interest, such as an antibody. In an embodiment, the targeting moiety is an antibody or a fragment thereof capable of binding to antigen, such as an intact antibody (e.g., a monoclonal antibody, a chimeric antibody and the like), a CDR region, a variable region, a Fab, a Fab', a F(ab)'₂, a single-chain Fv (scFv), a Fv fragment, a light chain of an antibody, a heavy chain of an antibody, a single-domain antibody, a diabody, and a linear antibody. Common targeting moieties are as described in Wu, A. M. and P. D. Senter. Nat Biotechnol 23(9): 1137-1146(2005).

The term "linear antibody" generally refers to the antibody described in Zapata et al., Protein Eng., 8(10): 1057-1062 (1995). Such an antibody comprises a pair of tandemly connected Fd segments (VH-CH1-VH-CH1) to form a pair of antigen binding regions together with the complementary light chain polypeptide. The linear antibody can be bi-specific or mono-specific.

Papain digests an antibody to generate two identical antigen binding fragments called "Fab" fragments and remaining "Fc" fragment. The Fab fragment is composed of the whole L chain along with the variable region domain (V_{H}) of H chain and the first constant domain (C_{H}1) of one heavy chain. Each Fab fragment is monovalent with respect to antigen binding, that is, it has one single antigen binding site. An antibody is subjected to trypsin treatment to generate a single large F(ab')₂fragment which roughly corresponds to two fab fragments linked by disulfide bond, has a bivalent antigen binding activity and still can cross-link with an antigen. The Fab' fragment, which is different from the Fab fragment, has several additional residues at the carboxyl terminal of the C_{H}1 domain, comprising one or more cysteines from the hinge region of an antibody.

"Fv" is a minimum antibody fragment containing a complete antigen recognizing and antigen binding site. This fragment is composed of a tightly non-covalent associated dimer of a heavy chain variable region domain and a light chain variable region domain. Six hypervariable loops (including three from each of H and L chains) are generated by folding both domains, providing amino acid residues for antigen binding and conferring antigen binding specificity to the antibody. A single variable domain (or half FV containing only three CDRs specific to the antigen) also has the capability of recognizing and binding to the antigen.

"Single-chain Fv", also abbreviated as "sFv" or "scFv, is an antibody fragment which comprises V_{H} and V_{L} antibody domains connected into a single polypeptide chain. Preferably, the sFv polypeptide further contains a polypeptide linker between the V_{H} and V_{L} domains, so that the sFv forms a structure necessary for antigen binding. For the review about sFv, see The Pharmacology of Monoclonal Antibodies, vol. 113, Rosenburg and Moore eds., Springer-Verlag, New York, pp. 269-315 (1994).

The term "diabody" refers to a small antibody fragment, which is prepared by constructing a sFv fragment between the V_{H} and V_{L} domains by a short linker (about 5-10 residues), thereby obtaining interchain rather than intrachain pairing of the V domains and generating a bivalent fragment, i.e., a fragment having two antigen binding sites. Bispecific diabody is a heterodimer of two "cross" sFv fragments, wherein the V_{H} and V_{L} domains of two antibodies are present in different polypeptide chains. See, for example, EP 404,097, WO 93/11161 and Hollinger et al., Proc. Natl. Acad. Sci. USA, 90:6444-6448 (1993).

The targeting moiety according to the invention can be linked to a biologically active moiety and/or a biologically inert moiety, directly covalently or indirectly via a linker. A method of linking a targeting moiety with a biologically active moiety or a biologically inert moiety is known in the art. Also, a person skilled in the art can determine according to the technical knowledge in the art that the targeting moiety is linked with the biologically active moiety or the biologically inert moiety directly covalently or via a linker (e.g., a linker set forth in SEQ ID NO: 7 or 8), thereby achieving the targeting function (e.g., specifically binding to a target of interest) of the targeting moiety and/or the therapeutic function of the biologically active moiety, for example, killing cancer cells, or the function of the biologically inert moiety, for example, shielding a target or degrading a target. There are various design methods for linkers. Common short peptide links can be found in Chen, X., et al. Adv Drug Deliv Rev 65(10): 1357-1369(2013). In an embodiment, the linker according to the invention is a short peptide of no more than 50 amino acids. In an embodiment, the linker according to the invention is a GS linker, for example, (GGGGS)ₙ (where n=1, 2, 3, 4, 5, 6, 7, 8, 9, 10), (Gly)ₙ linker (where n=1, 2, 3, 4, 5, 6, 7, 8, 9, 10), for example, (Gly)₆ or (Gly)₈, (EAAAK)ₙ linker (where n=1, 2, or 3), [A(EAAAK)ₙA]ₘ linker (where n=2, 3, 4, or 5, m=1 or 2), (Ala-Pro)ₙ linker (where n=5-17), or (XP)ₙ linker (where X represent any amino acid, preferably Ala, Lys or Glu; n=1, 2, 3, 4, 5, 6, 7, 8, 9, 10).

In an embodiment, the biologically active drug is an antibody of which the constant region is replaced by a moiety capable of exerting biological activity, therapeutic effect or pharmaceutical effect, wherein the active moiety capable of exerting biological activity, therapeutic effect or pharmaceutical effect may be selected from the group consisting of a cell toxin, a cytokine (such as IL-2, TNF-alpha, IL-12), a radioisotope, a chemotherapeutic agent, and a conjugated chemical drug.

The biologically inert moiety is selected from the group consisting of a constant region of an antibody such as IgG4 Fc, albumin, and polyethylene glycol.

In an embodiment, the targeting moiety is a variable region of an antibody, the biologically inert drug is a complete antibody with the constant region of the antibody, and the biologically active drug is an antibody of which the constant region is replaced by a moiety having pharmaceutical effect. The moiety having pharmaceutical effect may be selected from the group consisting of a cytokine (such as IL-2, TNF-alpha, IL-12), a radioisotope and a conjugated chemical drug.

In an embodiment, the targeting moiety is a variable region of an antibody, the biologically inert moiety is a constant region of IgG4 antibody, and the biologically active moiety is IL-2.

The disclosure provides a method for designing a drug for treating a disease related to ED-B, comprising:
- linking the antigen binding moiety of an ED-B specific antibody with a biologically active moiety, for example, directly covalently or indirectly via a linker (e.g., a linker as set forth in SEQ ID NO: 7 or 8); and/or
- linking the antigen binding moiety of an ED-B specific antibody with a biologically inert moiety, for example, directly covalently or indirectly via a linker (e.g., a linker as set forth in SEQ ID NO: 7 or 8).

Fibronectin (FN) is a multifunctional glycoprotein, expressed by epithelial cells, endothelial cells, fibroblasts, hepatocytes, decidual cells and extravillous trophoblasts, etc. The gene of FN, which is of about 75kb, contains about 50 exons and has a relative molecular weight of about 250kDa, is mainly composed of three types, type I, type II and type III, of homologous repetitive spherical domain units, and different domains are linked through a peptide chain which is sensitive to trypsin. Extradomain B (ED-B) is a complete domain comprising 91 amino acids, which is contained in the type III repetitive sequence of FN and encoded by a single exon. High expression of FN containing ED-B (FN(B+)) can be detected in primary lesions and metastatic sites of almost all of human solid cancers, and therefore, FN(B+) is used as a marker of tumor neovascularization (Carnemolla, Balza et al. (1989). J Cell Biol. 108: 1139-1148.).

"ED-B" refers to a protein or a homolog thereof containing an ED-B amino acid sequence wherein the ED-B amino acid sequence has, for example, an amino acid sequence set forth in SEQ ID NO: 10 or a sequence having at least 80%, 85%, 90%, 95%, 96%, 97, 98, 99% or higher sequence identity with SEQ ID NO: 10. The inventors found in the previous study that the blood contains ED-B proteins and that the ED-B protein level in the blood of a subject with tissue hyperplasia, for example, a tumor patient, is increased as compared with a health person or a subject not suffering from tumors (PCT/CN2015/094727).

Experimental data of phase I clinical drug metabolism of L19-IL2 indicated that: 1) when continuously administered in the same treatment cycle, the concentration of L19-IL2 in the blood (the area under the concentration-time curve per unit dosage, AUC/D) at the same dosage will be gradually reduced; 2) at the same doses of administration, the concentration of L19-IL2 in the blood (the area under the concentration-time curve per unit dosage, AUC/D) will be reduced with an increasing dosage (Johannsen, Spitaleri et al. (2010). Eur J Cancer. 46: 2926-2935). Without wishing to be bound by any theory, it can be speculated that: 1) when the drug is administered for the first time, there are a large quantity of ED-B antigens in blood, and L19-IL2 will bind to ED-B in blood; L19 antibodies are present in plasma for a long time for their high affinity, and the ED-B bound to the L19-IL2 will be metabolized together with L19-IL2 so as to be cleared from the blood. When the drug is administered again in the same treatment cycle, since the content of ED-B in the blood is reduced, the L19-IL2 bound thereto is reduced, and the free L19-IL2 may rapidly enter into solid tumor tissues. Therefore, the detected blood drug concentration (AUC/D) is lower than that in the previous drug administration. When the dosage is low, L19-IL2 fully binds to ED-B in the blood, and free L19-IL2 is less; with increasing dosage of L19-IL2, the binding thereof to the ED-B is close to saturation or supersaturated, and then free L19-IL2 is increased in the blood and can enter into solid tumor tissues more easily; and therefore, the detected L19-IL2 blood concentration (AUC/D) is reduced as the dosage is increased. Therefore, a large dosage of biologically inert L19 antibodies can be firstly administrated to oversaturatedly bind to the ED-B in the blood, and then a biologically active drug containing an L19 antibody recognition structure is administrated.

The disease related to ED-B according to the invention refers to a disease that the presence and/or expression of the ED-B in a diseased organ, tissue and/or cell.

The tumor according to the invention is an solid tumor, selected from, for example, teratoma, an upper digestive tract cancer, such as esophageal cancer, cardia cancer, laryngeal cancer, stomach cancer, head and neck cancer, liver cancer, biliary tract cancer, gallbladder cancer, colon cancer, duodenal cancer, lung cancer, bladder cancer, cervical cancer, ovarian cancer, endometrial cancer, breast cancer, melanoma, pancreatic cancer, kidney cancer and prostate cancer.

In an embodiment, the ED-B specific antibody includes, for example, CGS-1, CGS-2 (PCT/GB97/01412; Nissim, Hoogenboom et al. (1994).EMBO J. 13: 692-698), L19 (Pini, Viti et al. (1998). J Biol Chem. 273: 21769-21776), B5 (CN201480001324.5; WO2014/194784). In an embodiment, the ED-B specific antibody is L19 or B5 antibody. In an embodiment, the antigen binding moiety of the ED-B specific antibody is an intact antibody, a CDR region, a variable region, a Fab, a Fab', a F(ab)'₂, a single-chain Fv (scFv), a Fv fragment, a light chain of an antibody, a heavy chain of an antibody, a single-domain antibody, a diabody or a linear antibody.

The biologically active moiety is selected from is selected from TNF-alpha, IL-2 or IL-12.

The biologically inert moiety is selected from the group consisting of a constant region of antibody, albumin, and polyethylene glycol. In an embodiment, the biologically inert moiety is a constant region of antibody, for example, a constant region of IgG4 antibody (IgG4 Fc), such as a polypeptide set forth in SEQ ID NO: 4.

The disclosure also provides a development method for a biologically inert drug, and a corresponding biologically inert drug. The biologically inert drug according to the invention does not have the therapeutic effect of an active drug, or has a therapeutic effect but for different indications from the active drug. More particularly, the inert drug does not have the biological activity of the corresponding active drug.

The biologically inert drug can be obtained by modifying an active drug, to make it to just have the function of recognizing and binding to a target, the modification including but not limited to removal of the bioactive action structure, inactivation of the bioactive action site by mutation, replacement of the bioactive action site in the active drug, addition of a structure of inhibiting the bioactive action, such as shielding the active site of the drug through chemical modification, or fusion of a protein substantially having no biological activity to form steric hindrance.

The inert drug (drug chaperone) according to the invention needs to be used in combination with an active drug. An active drug and a drug chaperone targeting the same target can be prepared into a single preparation or a composition, or prepared into separate preparations or compositions, respectively. The drug chaperone is administered to bind to a specific target so as to block the target or partially block the target. The active drug is administered to target the unblocked target or a newly generated target, and thus the active drug can reach the lesion target in a more accurate and/or higher ratio. The drug chaperone is used to improve the safety, effectiveness and/or targeting of an existing active drug. For example, a biologically inert drug together with a biologically active drug can be prepared into a preparation for simultaneous administration. The biologically inert drug can also be an auxiliary material in a drug formula, which plays a role in competing with the biologically active drug to bind to free targets in the blood or other targets in normal tissues and also plays a role in protecting the stability of the biologically active drug.

A biologically inert drug can be obtained by altering, modifying or replacing a drug active structure as described above on the basis that only the targeting binding site of an active drug is retained, so that the existing active drug is changed into an inert drug. For example, the original activity of the active drug is reduced by more than 50%, more than 90%, more than 99%, and more than 99.9%. An inert drug and an active drug may have the same molecular structure, e.g. the ratio of the same structure in the molecular structure of the active drug is greater than 5%, 10%, 20%, 30%, 40%, 50%, 60%, 70, 80, and even more than 90%. In particular, the two drugs are mainly very similar in the molecular structures of the target binding sites, e.g. the similarity of the binding sites is up to 50%, 60%, 70%, 80%, 90%, 95%, and even 100%. The two drugs can bind to the same target molecule, and the positions to be bound in the target are close and even highly overlapped. Space steric hindrance is formed once the inert drug binds to the corresponding target, so that the active drug cannot bind to the same target as the inert drug.

A drug having biological inertia can recognize and bind to a specific target, and can shield a target and/or promote degradation of a target and/or maintain the stability of a biologically active drug. The inert moiety of a biologically inert drug (also referred to as a bio-inert molecule) can shield a target and/or promote degradation of a target, and can be, for example, an antibody constant region, albumin, or polyethylene glycol.

The drug having biological inertia can be a biological drug, including but not limited to a nucleic acid drug, a polypeptide drug, a protein drug, a chemical drug, and a biological drug-chemical drug conjugate. The drug having biological inertia includes but not limited to an antibody, a recombinant protein of antibody, an analogue or modifier of a drug having biological activity, and a biologically inert drug which is only identical to the target recognizing moiety of a drug having biological activity.

Alternatively, a drug having biological inertia and a drug having biological activity may not have the same chemical structure, or may not have the same mother nucleus. In an embodiment, the biologically inert drug is a nucleic acid ligand, and the biologically active drug is a protein. In an embodiment, the biologically inert drug can be based a constant region fragment of an antibody fused with mother-nucleus having targeting specificity, or a fused human albumin, a polyethylene glycol modification, a fused polypeptide fragment or a domain of a natural protein.

In an embodiment, the invention provides use of a drug chaperone of an ED-B protein related drug, for example, an antibody to ED-B protein, in the preparation of a kit, a cartridge or a pharmaceutical composition for treating a tumor in a mammal, for example, a human. In an embodiment, the drug chaperone involved in the invention is an antibody drug chaperone that targets ED-B of Fibronectin, the drug chaperone L19- or B5-IgG4 Fc which are formed by, based on L19 or B5 antibody as mother nucleus, keeping the variable regions of the L19 or B5 antibody and fusing with the antibody constant region fragment crystallizable (FC) protein, and may be combined with an L19 or B5 related drug for administration.

The method of developing a high-specificity drug system, a drug and a formula according to the invention is not only suitable for the development of anti-cancer drugs, but also suitable for the development of drugs for autoimmune diseases and other diseases.

The invention also provides a kit, a cartridge and a pharmaceutical composition produced by the above method and containing respective biologically active drugs and/or biologically inert drugs.

In one aspect, the invention also provides a composition comprising a biologically inert drug and a biologically active drug, wherein both the biologically inert drug and the biologically active drug have the same targeting moiety, wherein the biologically inert drug consists of a targeting moiety and a biologically inert moiety binding together through a covalent bond, and the biologically active drug consists of a targeting moiety and a biologically active moiety binding together through a covalent bond. In an embodiment, the targeting moieties of the biologically inert drug and the biologically active drug are an antibody or an antibody fragment.

As used herein, the term "kit" is interchangeable with "cartridge", which means a product comprising a combination of the biologically inert drug and the biologically active drug according to the invention or the pharmaceutical composition according to the invention, or a combination or a pharmaceutical composition of a biologically active drug and a biologically inert drug obtained by the method according to the disclosure, and optionally a drug administering tool and/or other desired reagents such as buffer, reconstitution solvent and the like.

In one aspect, the invention provides a drug system, a kit, a cartridge or a pharmaceutical composition, comprising a biologically inert drug and a biologically active drug according to the invention or a biologically active drug and a biologically inert drug obtained by the method according to the disclosure, and optionally, an administering tool, an administration instruction, a pharmaceutically acceptable carrier and/or an excipient, wherein the biologically inert drug and the biologically active drug are able to target the same target. In an embodiment, the biologically inert drug and the biologically active drug have the same targeting moiety.

The biologically active drug according to the invention can recognize and bind to a specific target and exert therapeutic effect. In an embodiment, the biologically active drug comprises a targeting moiety and a biologically active moiety, wherein the targeting moiety can recognize and bind to the target, and the biologically active moiety can exert bioactivity or therapeutic effect, such as an agent known to have specific action target, therapeutic effect, indications, etc.

In an embodiment, the targeting moiety is a molecule that specifically targets a target of interest, such as an antibody, a ligand, a receptor and the like or a part thereof. In an embodiment, the targeting moiety is selected from the group consisting of an intact antibody or a part or fragment thereof, such as a CDR region, a variable region, a Fab, a Fab', a F(ab)'₂, a single-chain Fv (scFv), a Fv fragment, a light chain of an antibody, a heavy chain of an antibody, a single-domain antibody, a diabody and a linear antibody.

The biologically active moiety is selected from IL-2, TNFα, and IL-12).

The biologically inert drug according to the invention can recognize and bind to a specific target, and shield the target, or promote the target to degrade or to be endocytosed by a cell, promote the target to enter into the digestive system, etc., thereby decreasing the targets to which the biologically active drug binds, including decreasing the targets distributed in normal tissues which the biologically active drug can bind to, and decreasing the targets distributed in blood which the biologically active drug can bind to. In particular, the biologically inert drug may block or partially block free protein targets in blood and protein targets distributed in normal tissues to reduce binding of the biologically active drug in non-target organs and non-target tissues, so that more biologically active drug can be distributed into a target organ and a target tissue, thereby improving the efficacy or the therapeutic effect of the biologically active drug on the lesion location and accordingly reducing the dosage of the biological active drug to reduce the toxic and side effects of the drug and improve the safety of the drug. Also, the biologically inert drug may play a role in protecting the stability of the biologically active drug. The biologically inert drug according to the invention does not have therapeutic effect, and needs to be used in combination with a biologically active drug. The biologically inert drug improves the metabolism and distribution of the biologically active drug, and is a chaperone for the biological active drug, which can also be referred to as a drug chaperone.

In an embodiment, the biologically inert drug comprises a targeting moiety and a biologically inert moiety. The targeting moiety can recognize and bind to a target, and the biologically inert moiety can shield the target or lead the target to degrade. In an embodiment, the biologically inert moiety is selected from a constant region of antibody, albumin, and polyethylene glycol. In an embodiment, the biologically inert moiety is the constant region of IgG4 antibody, for example, a polypeptide set forth in SEQ ID NO: 4.

In an embodiment, the invention provides a drug system, a kit, a cartridge or a pharmaceutical composition for treating ED-B related diseases such as tumors, comprising:
- a biologically active drug comprising a biologically active moiety linked to a targeting moiety targeting ED-B, for example, directly covalently or indirectly via a linker (e.g., a linker set forth in SEQ ID NO: 7 or 8), and
- a biologically inert drug comprising a biologically inert moiety linked to a targeting moiety targeting ED-B, for example, directly covalently or indirectly via a linker (e.g., a linker set forth in SEQ ID NO: 7 or 8).

The targeting moiety targeting ED-B is an EB-D antibody, such as CGS-1, CGS-2, L19 or B5 antibody, or an antigen binding fragment or part thereof, such as a CDR region, a variable region, a Fab, a Fab', a F(ab)'₂, a single-chain Fv (scFv), a Fv fragment, a light chain of an antibody, a heavy chain of an antibody, a single-domain antibody, a diabody and a linear antibody.

In an embodiment, the targeting moiety targeting ED-B is a single-chain Fv (scFv) of B5 antibody, wherein the light chain (VL) and the heavy chain (HL) of the B5 antibody are linked by an appropriate linker, such as (G₄S)₃ or a linker set forth in SEQ ID NO: 7 or 8.

In an embodiment, the biologically active moiety is IL-2 or an active fragment thereof, such as a polypeptide set forth in SEQ ID NO: 9.

In an embodiment, the biologically inert moiety is IgG4 Fc region, such as a polypeptide set forth in SEQ ID NO: 4.

In an embodiment, the biologically active drug is a polypeptide comprising an amino acid sequence set forth in SEQ ID NO: 6.

In an embodiment, the biologically inert drug is a polypeptide comprising an amino acid sequence set forth in SEQ ID NO: 5, or a dimer thereof.

In an embodiment, the biologically active drug and the biologically inert drug can be prepared as separate preparations or compositions, respectively, or mixed and prepared as a single preparation or composition. In the case of a single preparation or composition, the contents and ratio of the biologically active drug and the biologically inert drug in the preparation or composition can be determined by those skilled in the art according to actual requirements, for example, the molar ratio of the biologically active drug and the biologically inert drug can be from 10: 1 to 1: 10, such as 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10.

The "preparation" as described in the invention refers to a medicament which is produced according to a particular dose form requirement to meet the need of treating or preventing a disease and can be provided for administering to a subject. The preparation can contain the biologically active drug, the biologically inert chaperone according to the invention, and also other pharmaceutical adjuvants and tools.

The "adjuvant", i.e., pharmaceutical adjuvant, as described in the invention, refers to an agent that is used in producing a drug and formulating a prescription in addition to the active ingredients, already having been evaluated reasonably in safety and comprised in the pharmaceutical preparation, for the purpose of providing important functions such as solubilization, hydrotropy, sustained or controlled release and the like in addition to serving as an excipient or a carrier, or improving the stability.

The drug or the pharmaceutical composition according to the invention can be prepared into a dose form, such as tablets, powder, solution and the like, that is suitable for any appropriate administration route, such as intravenous, subcutaneous, parenteral, oral, intraperitoneal and the like. In an embodiment, the drug or the pharmaceutical composition according to the invention can be prepared into a form suitable for intravenous administration.

In an embodiment, the invention provides a kit, a cartridge or a pharmaceutical composition for treating a tumor in a mammal, for example, a human, comprising an effective amount of drug chaperone, for example, an antibody to ED-B protein, that can reduce the level of the ED-B protein, and an active drug targeting the ED-B protein, for example, an antibody drug of the ED-B protein.

As discussed herein, "reducing the level" refers to, as compared with a reference value, for example, a median or mean value observed in an untreated subject or before treatment with an inert drug, the level is reduced e.g. by at least 5%, at least 10%, at least 20%, at least 30%, at least 40%, at least 50%, at least 60%, at least 70%, at least 80%, at least 90%, or 100%.

The kit or the cartridge according to the invention may also comprise a container, label and/or instruction. A suitable container may include, for example, a bottle, a vial, a syringe, etc. Generally, the container may contain a composition for effective treatment of a condition and may have a sterile inlet (e.g., the container can be an intravenous injection solution bag). At least one active agent in the composition is the biologically active drug and/or the biologically inert drug according to the invention, or the pharmaceutical composition according to the invention, or a biologically active drug and/or a biologically inert drug or a pharmaceutical composition obtained by the method according to the disclosure. The label or the instruction may indicate particular conditions to be treated by the drug or the composition, and information about indications, usage, dosage, administration, contraindications and/or notes relating to the use of this product. In an embodiment, the instruction may indicate that the drug or the composition is used for treating ED-B related solid tumor.

In addition, the kit or the cartridge according to the invention may further comprise other containers that comprise a pharmaceutically acceptable buffer solution, such as bacteriostatic water for injection (BWFI), phosphate buffered saline, Ringer's solution, and glucose solution. It may further comprise other materials meeting commercial and user needs, including other buffers, diluents, filters, syringe needles and syringes.

In one aspect, the invention provides products for use in a method for treatment of a disease, comprising administering the biologically active drug and the biologically inert drug or the pharmaceutical composition according to the invention, or a biologically active drug and a biologically inert drug or a pharmaceutical composition obtained by the method according to the disclosure to a subject in need of treatment.

For example, one drug may be administered first for binding to a specific target to block or partially block the target, and the other drug may be administered later, so that the drug administered later can target to a newly generated target or an unblocked target. The drug administered first may only play a role in target blocking to prevent the drug administered later from acting on the same target. Also, the drug administered first may have other biological activities, but the drug administered later may have different pharmaceutical effects or have the pharmaceutical activity of different intensity.

The biologically active drug and the biologically inert drug can be administered simultaneously, for example, administered in the form of a single preparation, or administered sequentially at a particular time interval, such as 5 minutes, 10 minutes, 15 minutes, 20 minutes, 25 minutes, 30 minutes, 40 minutes, 50 minutes, 1 hour, 2 hours, 3 hours, 4 hours, 5 hours, 6 hours, 7 hours, 8 hours, 9 hours, 10 hours, 11 hours, 12 hours or longer. For example, the biologically active drug is administered first, and then the biologically inert drug is administered; alternatively, the biologically inert drug is administered first, and then the biologically active drug is administered.

In the case of sequential administration, the time interval can be determined according to the known technical knowledge in the art or empirically. For example, it would be within the scope of technical knowledge and ability of those skilled in the art to determine an appropriate time interval according to the pharmacodynamics, half-life, clearance rate and the like of the administered drug.

The drug or the pharmaceutical composition according to the invention can be administered by any suitable route, such as intravenously, subcutaneously, parenterally, oral, intraperitoneally and the like. The dosage and regimen of administration can be determined according to a physician's experience or relevant manuals. In an embodiment, the drug or the pharmaceutical composition according to the invention can be administered intravenously.

The dosage and/or ratio of the administered biologically active drug and biologically inert drug can be determined according to a physician's experience or relevant manuals. In an embodiment, the molar ratio of the biologically active drug to the biologically inert drug can be from 10:1 to 1:10, such as 10:1, 9:1, 8:1, 7:1, 6:1, 5:1, 4:1, 3:1, 2:1, 1:1, 1:2, 1:3, 1:4, 1:5, 1:6, 1:7, 1:8, 1:9 or 1:10.

In one aspect, the invention provides products for use in a method for treating a mammal (e.g., human) suffering from an ED-B related solid tumor, comprising administering to the animal a biologically active drug and a biologically inert drug or a pharmaceutical composition according to the invention, or a biologically active drug and a biologically inert drug or a pharmaceutical composition obtained by the method according to the disclosure, wherein the biologically active drug comprises a biologically active moiety linked to a targeting moiety targeting ED-B (e.g., directly covalently or indirectly via a linker), and the biologically inert drug comprises a biologically inert moiety linked to a targeting moiety targeting ED-B (e.g., directly covalently or indirectly via a linker).

The targeting moiety targeting ED-B is an EB-D antibody, for example, CGS-1, CGS-2, L19 or B5 antibody, or an antigen binding fragment thereof, such as a CDR region, a variable region, a Fab, a Fab', a F(ab)'₂, a single-chain Fv (scFv), a Fv fragment, a light chain of an antibody, a heavy chain of an antibody, a single-domain antibody, a diabody and a linear antibody.

In an embodiment, the targeting moiety targeting ED-B is a single-chain Fv (scFv) of B5 antibody, wherein the light chain (VL) and the heavy chain (HL) of the B5 antibody are linked by an appropriate linker, such as (G₄S)₃ or a linker set forth in SEQ ID NO: 7 or 8.

In an embodiment, the biologically active moiety is IL-2 or a fragment thereof, as set forth in SEQ ID NO: 9.

In an embodiment, the biologically inert moiety is IgG4 Fc region, as set forth in SEQ ID NO: 4.

In an embodiment, the biologically active drug is a polypeptide comprising an amino acid sequence set forth in SEQ ID NO: 6.

In an embodiment, the biologically inert drug is a polypeptide comprising an amino acid sequence set forth in SEQ ID NO: 5, or a dimer thereof.

In an embodiment, the drug or the pharmaceutical composition according to the invention is administered to the mammal intravenously. In an embodiment, the biologically inert drug and the biologically active drug are administered simultaneously, for example, in the form of a pharmaceutical composition comprising the biologically inert drug and the biologically active drug.

In an embodiment, the biologically inert drug is administered first to the mammal and then the biologically active drug is administered, for example, at an interval of 5 minutes, 10 minutes, 15 minutes, 20 minutes, 25 minutes, 30 minutes, 40 minutes, 50 minutes, 1 hour, 2 hours, 3 hours, 4 hours, 5 hours, 6 hours, 7 hours, 8 hours, 9 hours, 10 hours, 11 hours, 12 hours or longer.

### Examples

The disclosure provides an inventive drug system design method, a drug design method, and a pharmaceutical formulating method. In addition, the invention relates to a drug production. The invention will be further illustrated by the following examples, but any example or a combination thereof shall not be construed as limitations to the scope or implementation mode of the invention.

### Example 1: detection of ED-B protein in blood

### (A) Expression and purification of ED-B protein antibody

The DNA sequences of single-chain antibody (scFv) of B5 and L19 were synthesized, respectively (see patent application No. CN201480001324.5, Pini, Viti et al. (1998).J Biol Chem. 273: 21769-21776 and Borsi, Balza et al. (2002).Int J Cancer. 102: 75-85). A DNA sequence encoding a signal peptide was added to the 5'-terminal of the DNA sequence of each antibody, and a DNA sequence encoding IgG1 Fc tag was added to the 3' terminal thereof (Cao, Cao et al. (2009).Appl Biochem Biotechnol. 157: 562-574). The N-terminal and C-terminal of this sequence were digested by Nhe I and Not I restriction enzymes and cloned into pCI-neo vector to generate pCI-neo-B5-Fc vector and pCI-neo-L19-Fc vector. The construction and expression of a vector for an antibody protein may be referenced to (Borsi, Balza et al. (2002).Int J Cancer. 102: 75-85.).

The obtained vectors containing the antibody fusion protein were transfected to CHO-K1 cells by Lipofectamine^{®} 2000 of Invitrogen, after continuous selective culture using neo gene of pCI-neo plasmid vector in a G418-containing selective medium for 4 weeks (Davies and Jimenez (1980). Am J Trop Med Hyg. 29: 1089-1092.), clonal culturing was carried out by a limited dilution method, and the obtained single colony cell was continued to clonal culturing to obtain a stable strain.

The obtained cell strain was suspended and expressed by Hyclone CD4CHO. The antibody protein with the Fc tag was purified by using Protein A. The purity of the purified protein was detected by SDS-PAGE electrophoresis to obtain the purity of the sample, and the concentration of the sample was detected by an ultraviolet spectrophotometer.

### (B) Detection of ED-B in blood by ELISA

The blood of a healthy person and of a patient (suffering from nasopharyngeal carcinoma, esophageal cancer, stomach cancer, lung cancer, pancreatic cancer, hyperplasia of mammary glands, benign tumors) was taken and treated by sodium citrate anticoagulant prior to centrifuge for 2 minutes at 4°C and 3000 g, and the precipitates was discarded. This process was repeated once.

The ED-B protein in the blood samples was detected by sandwich ELISA using L19-IL2 (see Carnemolla, Borsi et al. (2002).Blood. 99: 1659-1665.) and B5-Fc antibodies.

In brief, the antibody L19-IL2 was diluted with a carbonate buffer solution having pH9.6 (Na₂CO₃ 1.59 g/L, NaHCOs 2.93 g/L) to the concentration of 2 ng/µl, and applied to coat ELISA plate at 100µl/well at 37°C for 2 hours. The coated ELISA plate was washed with PBST (NaCl 8 g/L, KCl 0.2 g/L, Na₂HPO₄ 1.42 g/L, KH₂PO₄ 0.27 g/L, pH=7.4, 0.5‰ Tween-20) for 3 times, and then blocked with PBS solution (NaCl 8 g/L, KCl 0.2 g/L, Na₂HPO₄ 1.42 g/L, KH₂PO₄ 0.27 g/L, pH=7.4) containing 4% BSA at 37°C for 1 hour. The ELISA plate was washed with PBST for 3 times.

A serum sample diluted by 50 folds with PBS (NaCl 8 g/L, KCl 0.2 g/L, Na₂HPO₄ 1.42 g/L, KH₂PO₄ 0.27 g/L, pH=7.4) was added to the sample wells of the ELISA plate at 100µl/well. Meanwhile, the BSA was used as negative control. The plate was incubated at 37°C for 1 hour. The plate was washed for 3 times with PBST, and the B5-Fc antibody was diluted to 2ng/µl by PBST, added to the sample wells of the plate at 100µl/well, and incubated at 37 °C for 1 hour. The plate was washed for 3 times with PBST, and added with HRP-labeled mouse anti-human IgG antibody (Boster BA1070) diluted by 5000 times with PBST, and incubated at 37°C for 1 hour. The plate was washed for 5 times with PBST, and a TMB developing solution was added to the sample wells at 100µl/well and incubated at room temperature for 5 minutes. The reaction was stopped using 2mol/L H₂SO₄ at 100µl/well. A plate reader was used for detection at dual-wavelength of OD450 nm and OD630 nm. The background value of the blank control well was deducted, thereby obtaining the detected light absorption value of the sample.

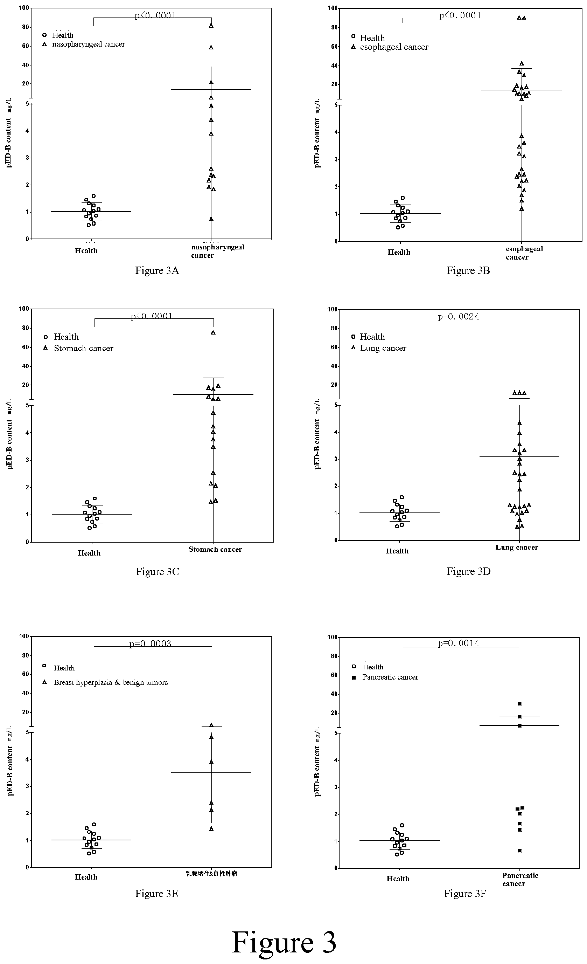

The results were shown in FIG. 3, indicating that the ED-B level in the blood of the solid tumor patient was remarkably increased, and the difference from the healthy person is statistically significant (Mann-Whitney test).

### Example 2: Design and expression of a biologically inert drug and a biologically active drug

### 1) Design and expression of a biologically inert drug B5-Fc fusion protein (SEQ ID NO: 5)

The heavy chain (VH) (SEQ ID NO: 1) and the light chain (VL) (SEQ ID NO: 2) of B5 antibody were linked with a long linking fragment (SEQ ID NO: 7) to form single-chain antibody B5-ScFv (SEQ ID NO: 3), and the rear end thereof was fused with Fc fragment (SEQ ID NO: 4) of antibody IgG4, and the hinge region of the Fc fragment was kept. That is, cysteines were retained so as to form a dimer and improve the flexibility of the protein (see patent publication WO 2014/194784 for the B5 antibody). The carboxyl terminal of the B5 antibody was linked to the hinge region sequence of the amino terminal of Fc by three alanine residues. After fusion, protein B5-Fc (SEQ ID NO: 5) was formed.

### 2) Design and expression of a biologically active drug B5-IL2 fusion protein (SEQ ID NO: 6)

The heavy chain (VH) (SEQ ID NO: 1) and the light chain (VL) (SEQ ID NO: 2) of B5 antibody were linked with a long linking fragment (SEQ ID NO: 7) to form single-chain antibody B5-ScFv (SEQ ID NO: 3), and the rear end thereof was fused with human interleukin 2 (IL2) (SEQ ID NO: 9), and the carboxyl terminal of the B5 antibody was linked with IL2 by a long linking fragment (SEQ ID NO: 8). After fusion, protein B5-IL2 (SEQ ID NO: 6) was formed.

### 3) Expression of B5-Fc and B5-IL2 fusion proteins

DNA fragments encoding the fusion protein of B5-Fc(SEQ ID NO: 5) and B5-IL2 (SEQ ID NO: 6) were linked into PCI-neo vectors, respectively. After a large amount of plasmids containing the antibody fusion protein encoding sequences was extracted, the plasmid DNA was transfected to CHO-K1 cells (purchased from China Center for Type Culture Collection) by using lipofectamine^{®} 2000 (Invitrogen). After continuous selective culture using neo gene of pCI-neo plasmid vector in a G418-containing selective DMEM/F12 medium (Thermo Scientific HyClone) for 4-8 weeks, clonal culturing was carried out by a limited dilution method, and the obtained single colony cells were continued to clonal culturing to obtain a stable strain.

The obtained cell strain was suspended and expressed after being subjected to adaptation by Hyclone SFM4CHO-Utility (Thermo Scientific HyClone). Verified by ELISA and SDS-PAGE experiment, the desired antibody proteins were obtained, and the yield of the antibody proteins was about 100 mg/L.

### Example 3: animal modeling, grouping and administration method

Preparation of solution: the B5-Fc ad B5-IL2 fusion proteins prepared in example 1 were prepared in PBS (NaCl 8g/L, KCl 0.2g/L, Na₂HPO₄ 1.42g/L, KH₂PO₄ 0.27g/L, pH=7.4) into 4 mg/ml solutions, respectively.

Establishment of a mouse transplantation tumor model: healthy Balb/c nu nude mice were fed to eight weeks old in a SPF-grade barrier system, and mouse teratoma cells (F9) were inoculated at the back of the mouse subcutaneously (Shanghai Fuxiang Bio-Technology Co., Ltd.) at each injection point with 2.5 × 10⁶ cells. Mice with a tumor volume of about 60 mm³ were grouped for experiments in day 6 after inoculation. Mice were grouped by 8 per group, and marked as group A, B, C, D and E. On day 6, 7 and 8 after inoculation, the drugs were administered through tail intravenous injection, respectively.

The group A was negative control group, and administered with normal saline twice a day at an interval of 6 hours, 50µl/mouse each time.

The group B was positive control group, and administered with B5-IL2 solution and normal saline each once every day, the normal saline at first, and the B5-IL2 at an interval of 6 hours, 50µl/mouse respectively.

The group C was administered B5-Fc solution first and B5-IL2 solution 6 hours later every day, 50µl/mouse respectively.

The group D was administered once with a mixed solution of B5-IL2 and B5-Fc in equal volume, 100µl/mouse.

The group E was control group, and administered with B5-Fc solution and normal saline each once every day, the B5-Fc solution at first, and then the normal saline at an interval of 6 hours, 50µl/mouse respectively.

On day 6 after the last administration, i.e. at day 14 after the subcutaneous inoculation modeling of the mice, the volumes were measured. The results were shown in the table 1: groups A and E, the negative control groups, had tumor volumes significantly larger than those of groups B, C and D, and the differences were statistically significant. The tumor volume of the group c or the group d was obviously smaller than that of the group B with statistical significance, so that the treatment effect of the groups C and D is better than that of the group B. No significant difference exists between the group C and the group D. No significant difference exists between the group A and the group E.

**Table 1**

| Groups | Group A | Group B | Group C | Group D | Group E |
|---|---|---|---|---|---|
| Tumor volume (mm³) | 1202.0±223.1 | 481.6±72.69 | 206.6±89.76 | 207.9±43.4 | 1076±183.3 |

## Claims

1. A kit for use in treating a solid tumor associated with ED-B in a subject, wherein the solid tumor associated with ED-B has presence and/or expression of ED-B,
the kit comprising a biologically inert drug and a biologically active drug, wherein
- the biologically active drug comprises a targeting moiety and a biologically active moiety;
- the biologically inert drug comprises a targeting moiety and a biologically inert moiety;
optionally the biologically inert drug and the biologically active drug have the same targeting moiety,
wherein the biologically inert drug and the biologically active drug are mixed and prepared into a single preparation or composition, or the biologically inert drug and the biologically active drug are prepared into separate preparations or compositions, respectively,
wherein the targeting moiety is an antibody or a binding fragment thereof against the Extra-domain B (ED-B) of fibronectin,
wherein the biologically active moiety is selected from TNF-alpha, IL-2 or IL-12,
wherein the biologically inert moiety is selected from the group consisting of an antibody constant region, albumin, and a polyethylene glycol,
and wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug.

2. A pharmaceutical composition for use in treating a solid tumor associated with ED-B in a subject, wherein the solid tumor associated with ED-B has presence and/or expression of ED-B,
the composition comprising a biologically inert drug, a biologically active drug, and optionally a pharmaceutically acceptable carrier and/or excipient, wherein
- the biologically active drug comprises a targeting moiety and a biologically active moiety;
- the biologically inert drug comprises a targeting moiety and a biologically inert moiety;
optionally the biologically inert drug and the biologically active drug have the same targeting moiety,
wherein the targeting moiety is an antibody or a binding fragment thereof against the Extra-domain B (ED-B) of fibronectin,
wherein the biologically active moiety is selected from TNF-alpha, IL-2 or IL-12,
wherein the biologically inert moiety is selected from the group consisting of an antibody constant region, albumin, and a polyethylene glycol,
and wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug.

3. The kit for use or the pharmaceutical composition for use according to any one of previous claims, wherein the biologically active moiety is cytokine IL-2 or a peptide set forth in SEQ ID NO: 9, and/or the biologically active drug is a polypeptide set forth in SEQ ID NO: 6.

4. A biologically inert drug for use in treating a solid tumor associated with ED-B in a subject, wherein the solid tumor associated with ED-B has presence and/or expression of ED-B,
the biologically inert drug comprising a targeting moiety targeting a target of interest and a biologically inert moiety, wherein the targeting moiety is an antibody or a binding fragment thereof against the Extra-domain B (ED-B) of fibronectin, and the biologically inert moiety is selected from the group consisting of an antibody constant region, albumin, and a polyethylene glycol,
wherein the treatment comprises administering a biologically active drug comprising a targeting moiety and a biologically active moiety,
and wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug,
wherein the targeting moiety of the biologically active drug is an antibody or a binding fragment thereof against the Extra-domain B (ED-B) of fibronectin, and
wherein the biologically active moiety of the biologically active drug is selected from TNF-alpha, IL-2 or IL-12.

5. The kit for use or the pharmaceutical composition for use or the biologically inert drug for use according to any one of previous claims, wherein the targeting moiety is selected from the group consisting of an intact antibody, an antibody CDR, an antibody variable region, a Fab fragment, a Fab' fragment, a F(ab)'₂ fragment, a single-chain FV (scFV), a FV fragment, a light chain of an antibody, a heavy chain of antibody, a single-domain antibody, a diabody and a linear antibody.

6. The kit for use or the pharmaceutical composition for use or the biologically inert drug for use according to any one of previous claims, wherein the biologically inert moiety is IgG4 Fc region.

7. The kit for use or the pharmaceutical composition for use or the biologically inert drug for use according to any one of previous claims, wherein the targeting moiety is selected from the group consisting of CGS-1, CGS-2, L19, B5 antibody and an ED-B binding fragment thereof, wherein the ED-B binding fragment is selected from the group consisting of an antibody CDR, an antibody variable region, a Fab fragment, a Fab' fragment, a F(ab)'₂ fragment, a single-chain FV (scFV), a FV fragment, a light chain of an antibody, a heavy chain of an antibody, a single-domain antibody, a diabody and a linear antibody.

8. The kit for use or the pharmaceutical composition for use or the biologically inert drug for use according to any one of previous claims, wherein the biologically inert moiety is a peptide set forth in SEQ ID NO: 4, and/or the biologically inert drug is a polypeptide set forth in SEQ ID NO: 5 or a dimer thereof.

9. The kit for use or the pharmaceutical composition for use or the biologically inert drug for use according to any one of previous claims, wherein the targeting moiety is a peptide set forth in SEQ ID NO: 3.

10. A biologically inert drug comprising a targeting moiety targeting a target of interest and a biologically inert moiety, wherein the targeting moiety is an antibody or a binding fragment thereof against the Extra-domain B (ED-B) of fibronectin, and the biologically inert moiety is selected from the group consisting of an antibody constant region, albumin, and a polyethylene glycol,
wherein the biologically inert moiety is a polypeptide set forth in SEQ ID NO: 4, and/or the biologically inert drug is a polypeptide set forth in SEQ ID NO: 5 or a dimer thereof,
wherein the biologically inert drug substantially has no biological activity relative to a biologically active drug, which means that the biologically inert drug does not have the pharmaceutical effect as the biologically active drug, or at the same administered dosage, its pharmaceutical effect may be neglected compared with the biologically active drug.

## Patentansprüche

1. Kit zur Verwendung bei der Behandlung eines mit ED-B assoziierten soliden Tumors bei einem Patienten, wobei der mit ED-B assoziierte solide Tumor das Vorhandensein und/oder die Expression von ED-B aufweist,
wobei das Kit ein biologisch inertes Medikament und ein biologisch aktives Medikament umfasst, wobei
- das biologisch aktive Medikament eine Targeting-Einheit und eine biologisch aktive Einheit umfasst;
- das biologisch inerte Medikament eine Targeting-Einheit und eine biologisch inerte Einheit umfasst;
wobei optional das biologisch inerte Medikament und das biologisch aktive Medikament dieselbe Targeting-Einheit haben,
wobei das biologisch inerte Medikament und das biologisch aktive Medikament gemischt und zu einer einzigen Zubereitung oder Zusammensetzung verarbeitet werden oder das biologisch inerte Medikament und das biologisch aktive Medikament jeweils zu separaten Zubereitungen oder Zusammensetzungen verarbeitet werden,
wobei die Targeting-Einheit ein Antikörper oder ein Bindungsfragment davon gegen die Extra-Domäne B (ED-B) von Fibronektin ist,
wobei die biologisch aktive Einheit aus TNF-alpha, IL-2 oder IL-12 ausgewählt ist, wobei die biologisch inerte Einheit aus der Gruppe ausgewählt ist, die aus einer konstanten Antikörperregion, Albumin und einem Polyethylenglykol besteht,
und wobei das biologisch inerte Medikament im Wesentlichen keine biologische Aktivität im Vergleich zu einem biologisch aktiven Medikament aufweist, was bedeutet, dass das biologisch inerte Medikament nicht die pharmazeutische Wirkung wie das biologisch aktive Medikament aufweist oder dass bei der gleichen verabreichten Dosierung seine pharmazeutische Wirkung im Vergleich zu dem biologisch aktiven Medikament vernachlässigt werden kann.

2. Pharmazeutische Zusammensetzung zur Verwendung bei der Behandlung eines mit ED-B assoziierten soliden Tumors bei einem Patienten, wobei der mit ED-B assoziierte solide Tumor das Vorhandensein und/oder die Expression von ED-B aufweist,
wobei die Zusammensetzung ein biologisch inertes Medikament, ein biologisch aktives Medikament und optional einen pharmazeutisch verträglichen Träger und/oder Hilfsstoff umfasst, wobei
- das biologisch aktive Medikament eine Targeting-Einheit und eine biologisch aktive Einheit umfasst;
- das biologisch inerte Medikament eine Targeting-Einheit und eine biologisch inerte Einheit umfasst;
wobei optional das biologisch inerte Medikament und das biologisch aktive Medikament dieselbe Targeting-Einheit haben,
wobei die Targeting-Einheit ein Antikörper oder ein Bindungsfragment davon gegen die Extra-Domäne B (ED-B) von Fibronektin ist,
wobei die biologisch aktive Einheit aus TNF-alpha, IL-2 oder IL-12 ausgewählt ist, wobei die biologisch inerte Einheit aus der Gruppe ausgewählt ist, die aus einer konstanten Antikörperregion, Albumin und einem Polyethylenglykol besteht,
und wobei das biologisch inerte Medikament im Wesentlichen keine biologische Aktivität im Vergleich zu einem biologisch aktiven Medikament aufweist, was bedeutet, dass das biologisch inerte Medikament nicht die pharmazeutische Wirkung wie das biologisch aktive Medikament aufweist oder dass bei der gleichen verabreichten Dosierung seine pharmazeutische Wirkung im Vergleich zu dem biologisch aktiven Medikament vernachlässigt werden kann.

3. Kit zur Verwendung oder pharmazeutische Zusammensetzung zur Verwendung nach einem der vorhergehenden Ansprüche, wobei die biologisch aktive Einheit Zytokin IL-2 oder ein Peptid ist, das in SEQ ID Nr: 9 dargelegt ist, und/oder das biologisch aktive Medikament ein Polypeptid ist, das in SEQ ID Nr: 6 dargelegt ist.

4. Biologisch inertes Medikament zur Verwendung bei der Behandlung eines mit ED-B assoziierten soliden Tumors bei einem Patienten, wobei der mit ED-B assoziierte solide Tumor das Vorhandensein und/oder die Expression von ED-B aufweist,
wobei das biologisch inerte Medikament eine Targeting-Einheit, die auf ein Ziel von Interesse abzielt, und eine biologisch inerte Einheit umfasst, wobei die Targeting-Einheit ein Antikörper oder ein Bindungsfragment davon gegen die Extra-Domäne B (ED-B) von Fibronektin ist und die biologisch inerte Einheit aus der Gruppe ausgewählt ist, die aus einer konstanten Antikörperregion, Albumin und einem Polyethylenglykol besteht,
wobei die Behandlung das Verabreichen eines biologisch aktiven Medikaments umfasst, das eine Targeting-Einheit und eine biologisch aktive Einheit umfasst,
und wobei das biologisch inerte Medikament im Wesentlichen keine biologische Aktivität im Vergleich zu einem biologisch aktiven Medikament aufweist, was bedeutet, dass das biologisch inerte Medikament nicht die pharmazeutische Wirkung wie das biologisch aktive Medikament aufweist oder dass bei der gleichen verabreichten Dosierung seine pharmazeutische Wirkung im Vergleich zu dem biologisch aktiven Medikament vernachlässigt werden kann,
wobei die Targeting-Einheit des biologisch aktiven Medikaments ein Antikörper oder ein Bindungsfragment davon gegen die Extra-Domäne B (ED-B) von Fibronektin ist, und
wobei die biologisch aktive Einheit des biologisch aktiven Medikaments aus TNF-alpha, IL-2 oder IL-12 ausgewählt ist.

5. Kit zur Verwendung oder pharmazeutische Zusammensetzung zur Verwendung oder biologisch inertes Medikament zur Verwendung nach einem der vorhergehenden Ansprüche, wobei die Targeting-Einheit aus der Gruppe ausgewählt ist, die aus Folgendem besteht: einem intakten Antikörper, einer Antikörper-CDR, einer variablen Antikörperregion, einem Fab-Fragment, einem Fab'-Fragment, einem F(ab)'₂-Fragment, einem einkettigen FV (scFV), einem FV-Fragment, einer leichten Antikörperkette, einer schweren Antikörperkette, einem Einzeldomänenantikörper, einem Diabody und einem linearen Antikörper.

6. Kit zur Verwendung oder pharmazeutische Zusammensetzung zur Verwendung oder biologisch inertes Medikament zur Verwendung nach einem der vorhergehenden Ansprüche, wobei die biologisch inerte Einheit die IgG4-Fc-Region ist.

7. Kit zur Verwendung oder pharmazeutische Zusammensetzung zur Verwendung oder biologisch inertes Medikament zur Verwendung nach einem der vorhergehenden Ansprüche, wobei die Targeting-Einheit aus der Gruppe ausgewählt ist, die aus CGS-1, CGS-2, L19, B5-Antikörper und einem ED-B-Bindungsfragment davon besteht, wobei das ED-B-Bindungsfragment aus der Gruppe ausgewählt ist, die aus Folgendem besteht: einer Antikörper-CDR, einer variablen Antikörperregion, einem Fab-Fragment, einem Fab'-Fragment, einem F(ab)'₂-Fragment, einem einkettigen FV (scFV), einem FV-Fragment, einer leichten Antikörperkette, einer schweren Antikörperkette, einem Einzeldomänenantikörper, einem Diabody und einem linearen Antikörper.

8. Kit zur Verwendung oder pharmazeutische Zusammensetzung zur Verwendung oder biologisch inertes Medikament zur Verwendung nach einem der vorhergehenden Ansprüche, wobei die biologisch inerte Einheit ein Peptid ist, das in SEQ ID Nr: 4 dargelegt ist, und/oder das biologisch inerte Medikament ein Polypeptid, das in SEQ ID Nr: 5 dargelegt ist, oder ein Dimer davon ist.

9. Kit zur Verwendung oder pharmazeutische Zusammensetzung zur Verwendung oder biologisch inertes Medikament zur Verwendung nach einem der vorhergehenden Ansprüche, wobei die Targeting-Einheit ein Peptid ist, das in SEQ ID Nr: 3 dargelegt ist.

10. Biologisch inertes Medikament, das eine Targeting-Einheit, die auf ein Ziel von Interesse abzielt, und eine biologisch inerte Einheit umfasst, wobei die Targeting-Einheit ein Antikörper oder ein Bindungsfragment davon gegen die Extra-Domäne B (ED-B) von Fibronektin ist und die biologisch inerte Einheit aus der Gruppe ausgewählt ist, die aus einer konstanten Antikörperregion, Albumin und einem Polyethylenglykol besteht,
wobei die biologisch inerte Einheit ein Polypeptid ist, das in SEQ ID Nr: 4 dargelegt ist, und/oder das biologisch inerte Medikament ein Polypeptid, das in SEQ ID Nr: 5 dargelegt ist, oder ein Dimer davon ist,
wobei das biologisch inerte Medikament im Wesentlichen keine biologische Aktivität im Vergleich zu einem biologisch aktiven Medikament aufweist, was bedeutet, dass das biologisch inerte Medikament nicht die pharmazeutische Wirkung wie das biologisch aktive Medikament aufweist oder dass bei der gleichen verabreichten Dosierung seine pharmazeutische Wirkung im Vergleich zu dem biologisch aktiven Medikament vernachlässigt werden kann.

## Revendications

1. Kit pour utilisation dans le traitement d'une tumeur solide associée à l'ED-B chez un sujet, où la tumeur solide associée à l'ED-B a une présence et/ou une expression de l'ED-B,
le kit comprenant un médicament biologiquement inerte et un médicament biologiquement actif, où
- le médicament biologiquement actif comprend une fraction de ciblage et une fraction biologiquement active ;
- le médicament biologiquement inerte comprend une fraction de ciblage et une fraction biologiquement inerte ;
facultativement, le médicament biologiquement inerte et le médicament biologiquement actif ont la même fraction de ciblage,
où le médicament biologiquement inerte et le médicament biologiquement actif sont mélangés et préparés en une seule préparation ou composition, ou le médicament biologiquement inerte et le médicament biologiquement actif sont préparés en préparations ou compositions séparées, respectivement,
où la fraction de ciblage est un anticorps ou un fragment de liaison de celui-ci contre l'extra-domaine B (ED-B) de la fibronectine,
où la fraction biologiquement active est sélectionnée parmi TNF-alpha, IL-2 ou IL-12,
où la fraction biologiquement inerte est sélectionnée parmi le groupe consistant en une région constante d'anticorps, albumine et un polyéthylène glycol,
et où le médicament biologiquement inerte n'a substantiellement aucune activité biologique par rapport à un médicament biologiquement actif, ce qui signifie que le médicament biologiquement inerte n'a pas l'effet pharmaceutique comme le médicament biologiquement actif, ou qu'au même dosage administré, son effet pharmaceutique peut être négligé par rapport au médicament biologiquement actif.

2. Composition pharmaceutique pour utilisation dans le traitement d'une tumeur solide associée à l'ED-B chez un sujet, où la tumeur solide associée à l'ED-B a une présence et/ou une expression de l'ED-B,
la composition comprenant un médicament biologiquement inerte, un médicament biologiquement actif et facultativement un support et/ou un excipient pharmaceutiquement acceptable, où
- le médicament biologiquement actif comprend une fraction de ciblage et une fraction biologiquement active ;
- le médicament biologiquement inerte comprend une fraction de ciblage et une fraction biologiquement inerte ;
facultativement, le médicament biologiquement inerte et le médicament biologiquement actif ont la même fraction de ciblage,
où la fraction de ciblage est un anticorps ou un fragment de liaison de celui-ci contre l'extra-domaine B (ED-B) de la fibronectine,
où la fraction biologiquement active est sélectionnée parmi TNF-alpha, IL-2 ou IL-12,
où la fraction biologiquement inerte est sélectionnée dans le groupe consistant en une région constante d'anticorps, albumine et un polyéthylène glycol,
et où le médicament biologiquement inerte n'a substantiellement aucune activité biologique par rapport à un médicament biologiquement actif, ce qui signifie que le médicament biologiquement inerte n'a pas l'effet pharmaceutique comme le médicament biologiquement actif, ou qu'au même dosage administré, son effet pharmaceutique peut être négligé par rapport au médicament biologiquement actif.

3. Kit pour utilisation ou la composition pharmaceutique pour utilisation selon l'une quelconque des revendications précédentes, où la fraction biologiquement active est cytokine IL-2 ou un peptide présenté dans SEQ ID NO : 9, et/ou le médicament biologiquement actif est un polypeptide présenté dans SEQ ID NO : 6.

4. Médicament biologiquement inerte pour utilisation dans le traitement d'une tumeur solide associée à l'ED-B chez un sujet, où la tumeur solide associée à l'ED-B a une présence et/ou une expression de l'ED-B,
le médicament biologiquement inerte comprend une fraction de ciblage ciblant une cible d'intérêt et une fraction biologiquement inerte, où la fraction de ciblage est un anticorps ou un fragment de liaison de celui-ci contre l'Extra-domaine B (ED-B) de la fibronectine, et la fraction biologiquement inerte est sélectionnée parmi le groupe consistant en une région constante d'anticorps, albumine et un polyéthylène glycol,
où le traitement comprend administrer un médicament biologiquement actif comprenant une fraction de ciblage et une fraction biologiquement active,
et où le médicament biologiquement inerte n'a substantiellement aucune activité biologique par rapport à un médicament biologiquement actif, ce qui signifie que le médicament biologiquement inerte n'a pas l'effet pharmaceutique comme le médicament biologiquement actif, ou qu'au même dosage administré, son effet pharmaceutique peut être négligé par rapport au médicament biologiquement actif,
où la fraction de ciblage du médicament biologiquement actif est un anticorps ou un fragment de liaison de celui-ci contre l'Extra-domaine B (ED-B) de la fibronectine, et
où la fraction biologiquement active du médicament biologiquement actif est sélectionnée parmi TNF-alpha, IL-2 ou IL-12.

5. Kit pour utilisation ou la composition pharmaceutique pour utilisation ou le médicament biologiquement inerte pour utilisation selon l'une quelconque des revendications précédentes, où la fraction de ciblage est sélectionnée parmi le groupe consistant en un anticorps intact, un anticorps CDR, une région variable d'anticorps, un fragment Fab, un fragment Fab', un fragment F(ab)'₂, un FV (scFV) à chaîne unique, un fragment FV, une chaîne légère d'un anticorps, une chaîne lourde d'anticorps, un anticorps à domaine unique, un diacorps et un anticorps linéaire.

6. Kit pour utilisation ou la composition pharmaceutique pour utilisation ou le médicament biologiquement inerte pour utilisation selon l'une quelconque des revendications précédentes, où la fraction biologiquement inerte est la région IgG4 Fc.

7. Kit pour utilisation ou la composition pharmaceutique pour utilisation ou le médicament biologiquement inerte pour utilisation selon l'une quelconque des revendications précédentes, ou la fraction de ciblage est sélectionnée parmi le groupe consistant en anticorps CGS-1, CGS-2, L19, B5 et un fragment de liaison ED-B de celui-ci, où le fragment de liaison ED-B est sélectionné parmi le groupe consistant en un anticorps CDR, une région variable d'anticorps, un fragment Fab, un fragment Fab', un fragment F(ab)'₂, un FV (scFV) à chaîne unique, un fragment FV, une chaîne légère d'un anticorps, une chaîne lourde d'un anticorps, un anticorps à domaine unique, un diacorps et un anticorps linéaire.

8. Kit pour utilisation ou la composition pharmaceutique pour utilisation ou le médicament biologiquement inerte pour utilisation selon l'une quelconque des revendications précédentes, où la fraction biologiquement inerte est un peptide présenté dans SEQ ID NO : 4, et/ou le médicament biologiquement inerte est un polypeptide présenté dans SEQ ID NO : 5 ou un dimère de celui-ci.

9. Kit pour utilisation ou la composition pharmaceutique pour utilisation ou le médicament biologiquement inerte pour utilisation selon l'une quelconque des revendications précédentes, où la fraction de ciblage est un peptide présenté dans SEQ ID NO : 3.

10. Médicament biologiquement inerte comprenant une fraction de ciblage ciblant une cible d'intérêt et une fraction biologiquement inerte, où la fraction de ciblage est un anticorps ou un fragment de liaison de celui-ci contre l'Extra-domaine B (ED-B) de la fibronectine, et la fraction biologiquement inerte est sélectionnée parmi le groupe consistant en une région constante d'anticorps, albumine et un polyéthylène glycol,
où la fraction biologiquement inerte est un polypeptide présenté dans SEQ ID NO : 4, et/ou le médicament biologiquement inerte est un polypeptide présenté dans SEQ ID NO : 5 ou un dimère de celui-ci,
où le médicament biologiquement inerte n'a substantiellement aucune activité biologique par rapport à un médicament biologiquement actif, ce qui signifie que le médicament biologiquement inerte n'a pas l'effet pharmaceutique comme le médicament biologiquement actif, ou qu'au même dosage administré, son effet pharmaceutique peut être négligé par rapport au médicament biologiquement actif.
